# EUROPEAN PATENT APPLICATION

(11) **EP 3 708 195 A1**
(43) Date of publication of application: **16.09.2020**
(21) Application number: 19162377.6
(22) Date of filing: 12.03.2019
(51) Int. Cl.: A61M 1/00, A61M 27/00

(54) **BODY FLUID DRAINAGE DEVICE AND APPLICATION THEREOF**

(71) Applicant: Yang, Shu-Hui, Taipei City 104 (TW); Kuo, Fan-Ching, Taipei City 104 (TW)
(72) Inventor: Yang, Shu-Hui, Taipei City 104 (TW); Kuo, Fan-Ching, Taipei City 104 (TW)
(74) Representative: Lang, Christian

(57) **Abstract**

The present invention discloses a body fluid drainage device, which is adapted for being mounted on a frame for draining body fluid from an affected portion. The body fluid drainage device includes a body fluid drainage member for draining the body fluid from the affected portion and a body fluid drainage monitoring member detachably coupled to the body fluid drainage member for monitoring the drainage condition, so as to allow medical personnel to directly and/or indirectly utilize the body fluid drainage monitoring member to monitor the drainage condition.

## Description

### BACKGROUND OF THE INVENTION

### Field of the Invention

The invention relates to a medical equipment field, and more particularly to a body fluid drainage device and applications thereof.

### Description of the Related Art

With the advancement of technology, medical diagnosis and treatment are becoming more diverse, and the medical measures required to deal with various diseases are becoming more and more complicated so that the work of medical personnel is more and more complicated and cumbersome. Particularly in a condition of insufficient medical manpower, a medical staff, such as a professional nurse, often needs to monitor several patient status data such as blood pressure, heartbeat, respiration, blood oxygen, etc. Since the disease and treatment methods are different, the status data of each patient to be monitored are also different. Even more, for some specific data, in the case of using traditional medical equipment, it may be necessary to rely on medical personnel to observe the scale on the medical device around the patient one by one to obtain the data, and this increase the physical, mental and spiritual burden of medical personnel. In this case, for medical personnel who are burdened with a variety of medical and nursing work, especially in the case of long hours of work, the workload is quite heavy, which makes them vulnerable to fatigue, difficulty in concentration, and poor eyesight, and thereby increasing the risk of negligence in monitoring or disposal due to adverse conditions such as poor mental health. Therefore, in order to reduce such risks, how to enable forefront medical personnel to monitor the status of multiple patients in an easier, faster, and more efficient manner has become an urgent problem to be solved.

In addition, various medical tests for patients often have follow-up clinical and academic research purposes, which means that the current measurement of various states of the patient can help the medical personnel to make appropriate diagnosis and selection at the right time. In addition to the treatment, the data can be collected as a reference for the diagnosis of the patient in the future, and more importantly, the data of the many patients collected can also be obtained by the researcher for relevant academic research. In the future, we will continue to help clinical staff improve related medical technology. In this case, based on the needs of academic use, how to ensure the consistency and accuracy of the data collected by different medical personnel at different times is a very important issue. Because only when accuracy and consistent performance are ensured, the analysis of the data can be more objective, or from a quantitative research perspective, reflect the truth.

In summary, the present inventors have made efforts to provide a simpler and more efficient solution for medical personnel and medical researchers in view of the above problems, thereby reducing the clinical workload and the risk of making mistakes and reducing the difficulty of academic research. And improve its quality and accuracy.

### BRIEF SUMMARY OF THE INVENTION

An object of the invention is to provide a body fluid drainage device and applications thereof. The body fluid drainage device includes a body fluid drainage member and a body fluid drainage monitoring member detachably coupled to the body fluid drainage member and configured to monitor body fluid drainage immediately so that medical personnel quickly, accurately, and efficiently grasp the condition of fluid drainage of one or more patients so as to respond appropriately, and thus improve the efficiency, effectiveness, and workload of medical personnel, thereby reducing the risk of errors other happened in related or other work of medical personnel.

Another object of the invention is to provide a body fluid drainage device and applications thereof, wherein the body fluid drainage monitoring member includes a host and a flow rate measuring module, wherein the host includes an interface module, wherein the interface module includes a display unit and a input unit for a user setting parameters directly and check the monitoring data, history data and related parameters.

Another object of the invention is to provide a body fluid drainage device and applications thereof, wherein the host further includes a wired communication module allowing a user to connect an external device to the body fluid drainage device or the body fluid drainage monitoring member through cables so as to control related parameters and obtain monitoring data, thus significantly improve the user's work efficiency, reduce their physical, eyesight, mental burden and the risk of mistakes due to excessive work load.

Another object of the invention is to provide a body fluid drainage device and applications thereof, wherein the host further includes a wireless communication module allowing a user to connect an external device to the body fluid drainage device or the body fluid drainage monitoring member wirelessly so as to control related parameters and obtain monitoring data, thus significantly improve the user's work efficiency, reduce their physical, eyesight, mental burden and the risk of mistakes due to excessive work load.

Another object of the invention is to provide a body fluid drainage device and applications thereof, wherein the host further includes a processing module for aiding the user to calculate specific data such as the calculation of flow rate of the body fluid and analysis of variation of the flow rate based on the flow velocity of the body fluid. The data are transmitted to the user through the interface module and/or the wired communication module and/or the wireless communication module.

Another object of the invention is to provide a body fluid drainage device and applications thereof, the host further includes a data storage module electrically connected to the processing module to allow the processing module to save original data and calculated data, and thus allow the user to obtain the data directly or indirectly.

Another object of the invention is to provide a body fluid drainage device and applications thereof, the host further includes an alarm module electrically connected to the processing module. The alarm module alarms the user or other persons when the processing module finds abnormal data according to preset data or data set by the user.

Another object of the invention is to provide a body fluid drainage device and applications thereof, the host further includes a fastening member configured to detachably fasten the host to a movable frame, patient bed or other object as to mount the body fluid device or body fluid monitoring member in an appropriate position.

Another object of the invention is to provide a body fluid drainage device and applications thereof, the flow rate measuring module includes a conduit fastening member and a flow rate measuring member. The conduit fastening member detachably fastens the body fluid drainage tube so that the flow rate measuring member measures the flow velocity of the body fluid in the body fluid drainage tube precisely.

Another object of the invention is to provide a body fluid drainage device and applications thereof, the flow rate measuring member includes a first supersonic transceiver and a second supersonic transceiver. The first supersonic transceiver faces the second supersonic transceiver with the body fluid drainage tube being disposed therebetween. The first supersonic transceiver and the second supersonic transceiver transmit supersonic waves to each other so that the signal received by the first supersonic transceiver and the second supersonic transceiver is amplified so as to precisely measure the flow velocity of body fluid of different viscosity and haze.

Another object of the invention is to provide a body fluid drainage device and applications thereof, the first supersonic transceiver and the second supersonic transceiver can automatically regulate the strength of the supersonic wave to correspond the body fluid of different supersonic wave decay rate so that the received signal strength is maintained constant to stably measure the flow velocity of different body fluid.

Another object of the present invention is to provide a body fluid drainage device and applications thereof, the body fluid drainage device includes an body fluid drainage tube, a main board, a collecting member, and an liquid storage member, wherein the main board includes a board body having a first through hole for the body fluid drainage tube extending therethrough from a back side to a front side of the board body, thereby being connected to the collecting member movably fixed to the front surface of the plate body. The flow rate measuring module is detachably fixed to a portion of the body fluid drainage tube on the rear side of the board body to monitor the liquid flow rate in the tube, thereby avoiding the interference between the operation of the flow rate measuring module and drainage arrangement of the body fluid drainage tube on the front side of the board body and the adjustment and observation of the collecting element.

Another object of the present invention is to provide a body fluid drainage device and applications thereof, wherein the board body further has a second through hole and a third through hole, wherein the body fluid drainage tube comprises a first drainage tube, a switching member and a second drainage tube, wherein the switching member has a first end and a second end, wherein the first drainage tube extends from the back side to the front side of the board body through the first through hole and thereby being connected to the first end of the switching member, wherein the second drainage tube is connected to the second end of the switching member and extends through the second through hole to the back side of the board body and extends through the third through hole back to the front side of the board body, wherein the flow rate measuring module is detachably fixed to the portion of the body fluid drainage tube on the back side of the board body to monitor the flow rate in the tube, thereby avoiding the interference between the operation of the flow rate measuring module and drainage arrangement of the body fluid drainage tube on the front side of the board body and adjustment and observation of the collecting member, and the body fluid drainage tube can be more stably fixed to the board body.

Another object of the invention is to provide a body fluid drainage method, and the method includes the following steps: mounting a body fluid drainage member to a frame; coupling a body fluid drainage monitoring member to the body fluid drainage member; connecting the body fluid drainage member to an affected portion to drain body fluid; and monitoring body fluid drainage through the body fluid drainage monitoring member. The body fluid drainage device carries out the body fluid drainage and performs one or more of the described technical effects.

To solve the mentioned problems, the invention provides a body fluid drainage device adapted to installation on a frame for draining body fluid of an affected portion. The body fluid drainage device includes a body fluid drainage member configured to drain body fluid of the affected portion; and a body fluid drainage monitoring member detachably coupled to the body fluid drainage member and configured to monitor body fluid drainage.

According to an exemplary embodiment, the body fluid drainage member includes a body fluid drainage tube, and the body fluid drainage monitoring member is detachably coupled to the body fluid drainage tube and configured to monitor the body fluid drainage.

According to an exemplary embodiment, the body fluid drainage member further includes a main board and a collecting member movably disposed on the main board, wherein the body fluid drainage tube is connected to the collecting member for draining the body fluid to the collecting member, wherein the main board is adapted to installation on the frame near the affected portion so that the collecting member is movably disposed at a height corresponding to the affected portion.

According to an exemplary embodiment, the body fluid drainage tube includes a first drainage tube connected to the affected portion, a switching member and a second drainage tube, wherein the switching member has a first end connected to the first drainage tube, a second end connected to the second drainage tube, a third end adapted to connection of an external equipment and a switch configured to switch connection between the first end, the second end and the third end or between any two of the first end, the second end and the third end.

According to an exemplary embodiment, the main board includes a board body, a hanging member connected to the board body and configured to hang on the frame near the affected portion and a first movably-fastening member configured to movably fasten the collecting member, wherein the board body has a front surface, a back surface and a scale formed on the front surface and configured to indicate a position of the collecting member, wherein the body fluid member further comprises a body fluid storage member detachably disposed on the board body and connected to the colleting member for storing and depositing the body fluid.

According to an exemplary embodiment, the collecting member includes a main body configured to collect body fluid, an one-way valve configured to purge gas in the main body and a second movably fastening member connected to the main body and configured to movably coupled to the first movably fastening member so as to movably fasten the collecting member to the board body, wherein the collecting member has a body fluid inlet connected to the body fluid drainage tube, an exit connected to the one-way valve and a body fluid outlet, wherein the collecting member further comprises a body fluid outflow tube connected to the body fluid outlet and the body fluid storage member.

According to an exemplary embodiment, the board body includes at least one or more through holes through which the body fluid drainage tube extends so as to be arranged on a front side and a back side of the board body.

According to an exemplary embodiment, the body fluid drainage monitoring member includes a host and a flow rate measuring module connected to the host, wherein the flow rate measuring module is detachably coupled to the body fluid drainage tube for monitoring the body fluid drainage.

According to an exemplary embodiment, the host includes a power supply configured to supply electrical power, a processing module electrically connected to the power supply for calculation, a data storage module electrically connected to the processing module for storing data, and an interface module electrically connected to the processing module for inputting and outputting data.

According to an exemplary embodiment, the host further includes a fastening member configured to detachably fasten the host to the frame near the affected portion.

According to an exemplary embodiment, the host further includes an alarm module configured to alarm according to a control of the processing module.

According to an exemplary embodiment, the host further includes a wired communication module electrically connected to the processing module for wired communication.

According to an exemplary embodiment, the host further includes a wireless communication module electrically connected to the processing module for wireless communication.

According to an exemplary embodiment, the flow rate measuring module includes a conduit fastening member configured to detachably coupled to the body fluid drainage tube and a flow rate measuring member disposed in the conduit fastening member, wherein the flow rate measuring member includes a first supersonic transceiver and a second supersonic transceiver corresponding to the first supersonic transceiver, the first supersonic transceiver and the second supersonic transceiver transmit supersonic waves to measure a flow rate of the body fluid in the body fluid drainage tube.

According to another aspect of the invention, the invention further provides a body fluid draining method includes the following steps: (a) mounting a body fluid drainage member to a frame; (b) coupling a body fluid drainage monitoring member to the body fluid drainage member; (c) connecting the body fluid drainage member to an affected portion to drain body fluid; and (d) monitoring body fluid drainage through the body fluid drainage monitoring member.

According to an exemplary embodiment, the step (d) includes the following steps: (d1) connecting the body fluid drainage monitoring member to a terminal; and (d2) monitoring the body fluid drainage through the terminal.

### BRIEF DESCRIPTION OF THE DRAWINGS

Fig. 1A is a schematic view of an embodiment of a body fluid drainage device of the invention;
Fig. 1B is a schematic view of an alternative embodiment of a main board of a body fluid drainage member of a body fluid drainage device of the invention;
Fig. 2 is a schematic view of an embodiment of a flow rate measuring module of a body fluid drainage monitoring member of a body fluid drainage device of the invention;
Fig. 3 is a cross-sectional view of an embodiment of a flow rate measuring module of a body fluid drainage monitoring member of a body fluid drainage device of the invention;
Fig. 4 is a schematic view of an embodiment of an interface module of a host of a body fluid drainage monitoring member of a body fluid drainage device of the invention;
Fig. 5 is a schematic view of an alternative embodiment of an interface module of a host of a body fluid drainage monitoring member of a body fluid drainage device of the invention;
Fig. 6 is a block diagram of a host of a body fluid drainage monitoring member of a body fluid drainage device of the invention;
Fig. 7 is a schematic view of another embodiment of a body fluid drainage device of the invention;
Fig. 8 is a flow chart of an embodiment of a body fluid draining method of the invention; and
Fig. 9 is a flow chart of another embodiment of a body fluid draining method of the invention.

### DETAILED DESCRIPTION OF THE INVENTION

The embodiments of the present invention are described below by way of specific embodiments, and those skilled in the art can easily appreciate other advantages and effects of the present invention from the disclosure herein.

The following embodiments are described with reference to the drawings, and it should be noted that the following drawings are simplified schematic figures, and only basic concepts of the invention are illustrated in a schematic manner. The structure related to the invention is only schematically illustrated in the drawings and not drawn according to the number, shape and size of the units in actual implementation. The type, quantity and proportion of each unit in actual implementation are not limited to the drawings and can be modified according to actual design and requirements.

### Camera Module and Electromagnetic Shielding Member and Assembly Method Thereof and Applications

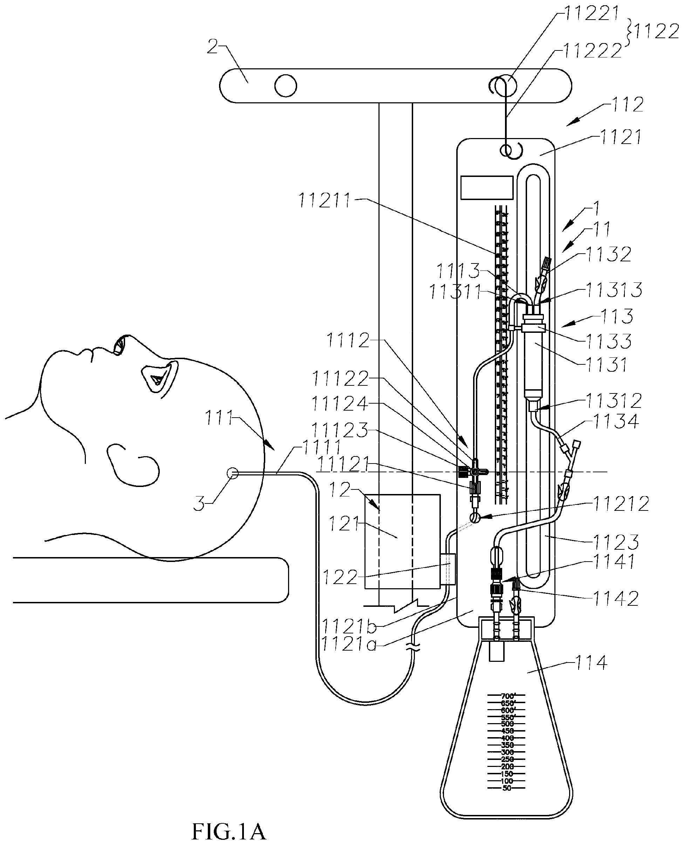

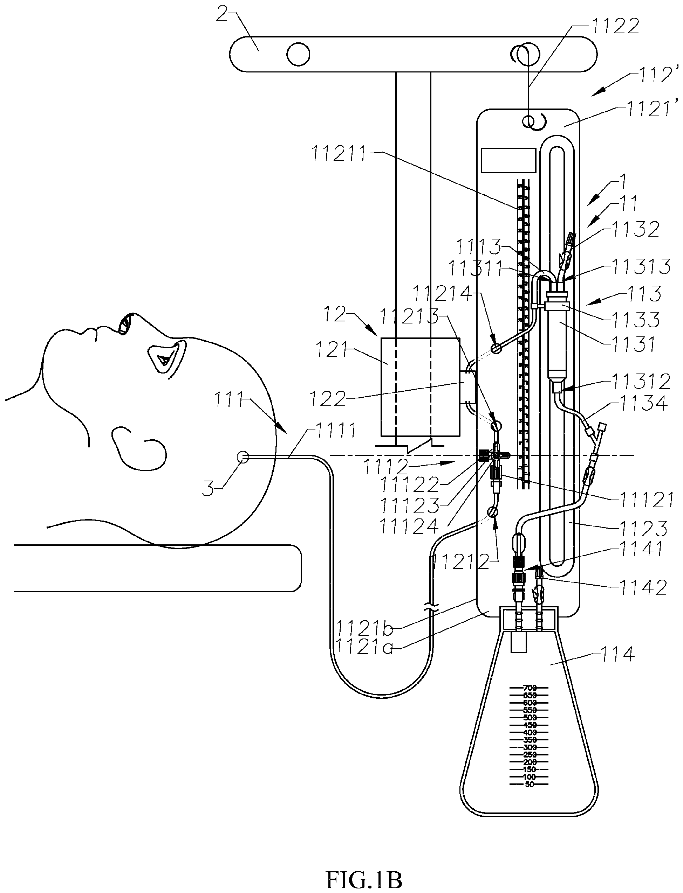

As shown in FIG. 1, a body fluid drainage device 1 according to a first preferred embodiment of the present invention is adapted to be disposed on a frame 2 for draining body fluids of an affected portion 3, wherein the body fluid drainage device 1 includes a body fluid drainage member 11 and a body fluid drainage monitoring member 12. The body fluid drainage member 11 is mainly used for draining body fluid of the affected portion, and the body fluid drainage monitoring member 12 is detachably coupled to the body fluid drainage member 11 for monitoring the drainage condition thereof. Here, the frame 2 as a workpiece refers to any object that is fixed or movable for the body fluid drainage device 1 to be mounted or movably fixed thereto, preferably implemented as a stainless tubular frame having a bottom with multiple claws and wheels. but can also be implemented as other objects having functions similar to that described above, such as bed rails, rails fixed to wall floors or ceilings, and the like, wherein the scope of the present invention is not limited to the aforementioned examples. Secondly, the affected portion 3 as a workpiece refers to a patient, such as a person or other living organisms, etc., a portion of the body that having fluid to be drained, or a portion that is subjected to drainage of body fluids, such as a lesion in the brain but needed to be drained from somewhere on an outer side of his/her head. That is to say, the affected portion 3 is not necessarily equivalent to the site where the lesion occurs and may also be a site for performing drainage to achieve therapeutic, support, improvement or other treatment effects, wherein the scope of the present invention is not limited thereto. Furthermore, the body fluids include cerebrospinal fluid, blood, lymph, tissue fluid, sputum, urine, and other secretions or other fluids produced from the patient himself and/or in vitro. The flow condition is preferably a flow rate when body fluid flows through a body fluid drainage tube 111 of the body fluid drainage member 11, thereby multiplying the measured flow rate by a known, preset or additional set diameter of the body fluid drainage tube 111. The flow rate per preset or additional set unit time is calculated. The drainage condition can also include the condition whether the flow of the body fluid is still or countercurrent, or the condition whether the body fluid drainage tube 111 is fully filled, the condition whether the body fluid is too rich or too thin, whether there is foreign matter and the level of the aforementioned conditions, etc., wherein the scope of the present invention is not limited thereto.

Further, according to the embodiment, the body fluid drainage member 11 includes a body fluid drainage tube 111, a main board 112, a collecting member 113 movably mounted to the main board 112 and a body fluid storage member 114. The body fluid drainage tube 111 is connected to the collecting member 113 to drain the body fluid of the affected portion to the collecting member 113. The main board 112 is adapted to be disposed on the frame 2 located near the affected portion 3 such that the height of the main board 112 relative to the affected portion is fixed to allow the collecting element 113 to be movably fixed to the specific height of the affected portion through the main board 12 for the medical staff to set and adjust the drainage pressure by adjusting the height at which the collecting member 113 is movably fixed to the main board 112. The body fluid drainage tube 111 includes a first drainage tube 1111, a switching member 1112, and a second drainage tube 1113. The body fluid drainage tube 111 is implemented as a pipeline connected to the affected portion for draining body fluid, wherein the first drainage tube 1111 is connected to the affected part to introduce body fluid in a manner that the first drainage tube 1111 is directly inserted into the affected portion, inserted into the affected portion through a joint, or connected to another drainage tube or the like located at the affected portion, wherein the specific practice of the invention referred to as connecting the affected portion or drainage of the body fluid from the affected portion is not limited to the aforementioned examples. The switching member 1112 includes a first end 11121 connected to the first drainage tube 1111 and a second end 11122 connected to the second drainage tube 1113 and a third end 11123 suitable for connecting an external equipment. And a switch 11124 for switching the first end 11121, the second end 11122, and the third end 11123 or any two of the first end 11121, the second end 11122, and the third end 11123. The switching member 1112 is mainly used for the medical personnel to switch on or off the drainage between the first end 11121 and the second end 11122 according to requirements, to bypass the drainage through the third end 11123, to add medicine, to access other equipment, to replace or for other disposal purposes.

According to the above first preferred embodiment of the present invention, the body fluid drainage monitoring member 12 is detachably coupled to the first drainage tube 1111 of the body fluid drainage tube 111 to monitor the drainage condition, however, the persons who are skilled in the art should understand that the body fluid drainage monitoring member 12 can also be detachably coupled to the second drainage tube 1113 of the body fluid drainage tube 111 to monitor the drainage condition and achieve the same technical effect, and thus the position at which the body fluid drainage monitor 12 of the present invention is detachably coupled to the body fluid drainage tube 111 is not limited.

Further, the main board 112 includes a board body 1121, a hanging member 1122 connected to the board body 1121, and a first movably fastening member 1123 disposed on the board body 1121. The hanging member 1122 is adapted to be hung from the frame 2 located near the affected portion to hang the main board 112. In this embodiment, the hanging member 1122 is implemented as a hole 11221 formed on the board body 1121 and a separate S-shaped hook 11222. The hook can pass through the hole to hook the board body 1121 and then further hooks a hole on the frame 2 which is implemented as a drip stand so as to hang the main board 112. However, those skilled in the art should understand that the hanging member 1122 is also used as any other structure that can hang the main board 112 in practical applications, for example, fixed to the board body 1121 through a structure of claw type and can be fixed to the bracket 2, such as in the form of a fastener, a rope, a magnetic member, or a specific shape of the board body 1121 enabling the board body 1121 to be hung on the frame 2 and the like. The scope of the present invention is not limited thereto.

Further, as shown in FIG. 1A, according to the aforementioned first preferred embodiment of the present invention, the board body 1121 has a scale 11211 and a first through hole 11212 for the body fluid drainage tube 111 extending from a back side 1121b to a front side 1121a of the board body 1121 so as to connect to the collecting member 113 movably fastened to a front side 1121a of the board body 1121. The collecting member 113 includes a main body 1131 for collecting body fluid, an one-way discharge valve 1132 for discharging the gas in the collecting member body 1131 in one direction, and a second movable fixing member 1133 connected to the collecting member body 1131 and adapted to be movably coupled to the first movably fastening member 1123 to movably fasten the collecting member 113 to the board body 1121 and a body fluid outflow pipe 1134, wherein the collecting member body 1131 has a body fluid inlet 11311 connected to the body fluid drainage tube 111, the body fluid outlet 11312, and an exit 11313 connected to the one-way discharge valve 1132. The body fluid storage element 114 has a body fluid storage member opening 1141 and a body fluid storage member one-way discharge valve 1142 for discharging the gas in the body fluid storage element 114 in one direction, wherein the body fluid outflow tube 1134 is connected to the body fluid outlet 11312 and the body fluid storage element opening 1141 of the body fluid storage element 114 respectively.

In this embodiment, the first movably fastening member 1123 disposed on the board body 1121 is implemented as an elongated hole; the second movably fastening member 1133 of the collecting member 113 is implemented as a lock fixed to the collecting member body 1131 so as to be moved upwards and downwards along the first movably fastening member 1123 when the lock is released, and then be locked when it is moved to a designated position to the collecting element body 1131 so as to be movably fixed to a specific height on the board body 1121. However, it should be understood by those skilled in the art that, in practical applications, the first movably fastening member 1123 and the second movably fastening member 1133 may also be implemented as other structures to achieve the same or similar effects, for example, a jaw structure is movably fixed on the board body 1121, a rivet and a hole fixed to each other, magnetic members fixed to each other by attractive force, or a rope structure tied to each other, and the like. The scope of the present invention is not limit thereto.

Further, the board body 1121 has a front side 1121a and a back side 1121b, wherein the scale 11211 is disposed on the front surface. The scale 11211 is mainly used for a medical personnel or other users to refer to the height of the hanging member 1122 relative to the affected portion and to adjust of the collecting member 113 to be movably fixed to a height of the main board 112. The medical personnel or other users can first align the zero point of the scale 11211 with the affected portion by adjusting the hanging element 1122, the frame 2, the position of the patient or the affected portion, and afterwards adjusting the position of the body fluid inlet 11311 of the collecting member body 1131 of the collecting element 113 through the first movably fastening member 1123 and the second movably fastening member 1133 to align the position of the scale 11211 so that the height of the collecting element 113 on the board body 1121 of the main board 112 is adjusted to adjust the height of the body fluid inlet 11311 relative to the affected portion for adjusting the drainage pressure.

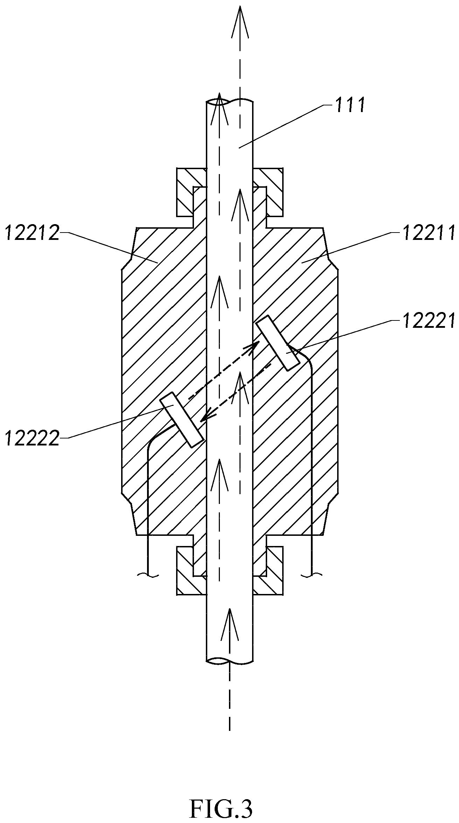

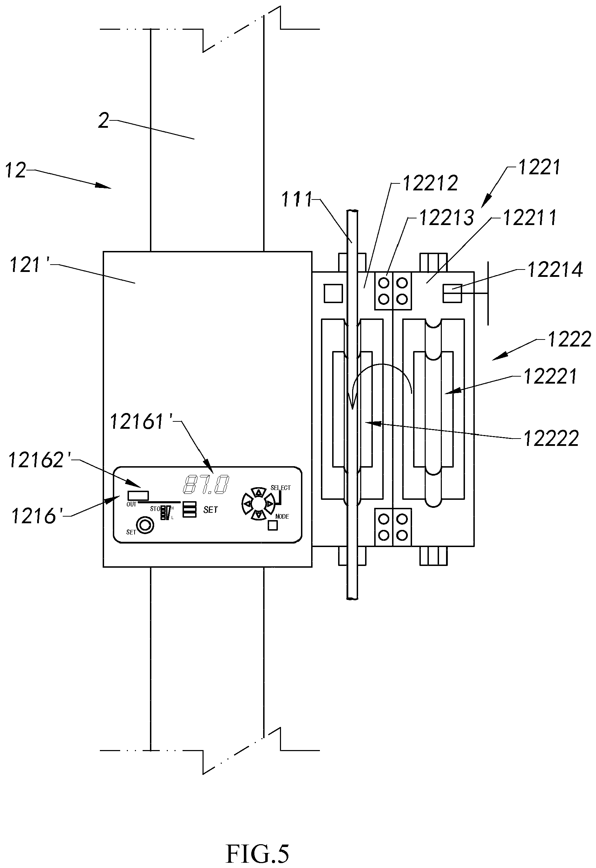

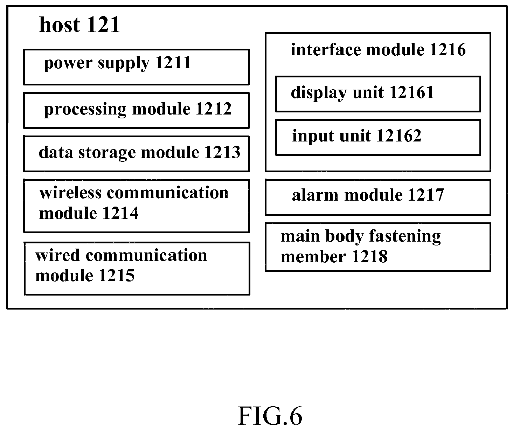

Referring to FIGs. 1-6, the body fluid drainage monitoring member 12 according to the aforementioned first preferred embodiment of the present invention includes a host 121 and a flow rate measuring module 122 electrically connected to the host 121, wherein the flow rate measuring module 122 is detachably coupled to the body fluid drainage tube 111 for monitoring the drainage condition. In other words, the flow rate measuring module 122 is mainly used to detect the drainage condition and transmit data to the host 121 for related applications, such as calculation, storage, transmission, and the like. The host 121 includes a power supply 1211 for providing power, a processing module 1212 electrically connected to the power supply 1211 for calculating, a data storage module 1213 electrically connected to the processing module 1212 for storing data, a wireless communication module 1214 electrically connected to the processing module 1212 for wireless communication, and a wired communication module 1215 for wired communication, an interface module 1216 electrically connected to the processing module 1212 for outputting and inputting information, and an alarm module 1217 electrically connected to the processing module 1212 for issuing an alarm according to the processing module's control. The processing module 1212 enables the host 121 to have a computing capability to execute a plurality of applications and control the flow rate measuring module 122 electrically connected to the host 121. According to preset parameters or parameters through the interface module 1216, the wireless communication module 1214, and/or the wired communication module 1215 that is, when the flow rate measuring module 122 detects a drainage condition of a specific level or property, the alarm module 1217 issues one or more specific alerts, such as sound, light, vibration, electronic signals, or other means, or alerts thereof may also be sent through the interface module 1216, the wireless communication module 1214, and/or the wired communication module 1215. In addition, the host 121 further includes a main body fastening member 1218 adapted to detachably fix the main body 121 to the bracket 2 located near the affected part 3. Further, the interface module 1216 includes a display unit 12161 and an input unit 12162. According to this embodiment, the display unit 12161 is implemented as a liquid crystal display for outputting information in a display manner for the user to directly obtain the specific or important information from the interface module 1216 of the host 121 so that users can complete some procedures intuitively and easily such as operation, setting parameters, viewing device status, troubleshooting, observing drainage condition, and so on. The input unit 12162 is implemented as a keyboard group composed of a plurality of different keys for the user directly and easily inputting information and instructions on the interface module 1216 of the host 121, so that the user can complete some procedures intuitively and simply, such as operation of several functions, setting parameters, viewing device status, troubleshooting, observing drainage condition, and the like. It should be noted that, according to other embodiments or in practical applications, the interface module 1216 may also be implemented as a touch screen to serve as the display unit 12161 and the input unit 12162, or may also be implemented as or have other input and output interfaces, such as gestures, vibration, remote control, wire control, sensing, voice control, and broadcast output device, etc., and the specific embodiments thereof and the scope of the present invention are not limited thereto.

In addition, the flow rate measuring module 122 is detachably fixed to the body fluid drainage tube 111 at a portion of the body fluid drainage tube 111 on the back side 1121b of the board body 1121 to monitor the fluid flow rate in the tube. Therefore, the fixation and operation of the flow detecting module 122 are prevented from interfering with the drainage arrangement of the body fluid drainage tube 111 on the front side 1121a of the board body 1121 and the adjustment and observation of the collecting member 113.

Referring to FIGs. 1A-1B, the main board 112 according to the above first preferred embodiment of the present invention includes a board body 1121, a hanging member 1122 coupled to the board body 1121, and a first movably fastening member 1123 disposed on the board body 1121, wherein the hanging member 1122 is adapted to be hung on the frame 2 located near the affected portion to suspend the main board 112. Further, as shown in FIG. 1B, according to the main board 112' of the aforementioned first preferred embodiment of the present invention, the main board 112' includes a board body 1121', a hanging member 1122 connected to the board body 1121' and the first movably fastening member 1123 disposed on the board body 1121, wherein the hanging member 1122 is adapted to be hung on the frame 2 located near the affected portion to hang the main board 112', wherein the board body 1121' has the scale 11211 and at least three through holes, a first through hole 11212, a second through hole 11213, and a third through hole 11214. The body fluid drainage tube 111 includes the first drainage tube 1111, the switching member 1112, and the second drainage tube 1113, wherein the switching member 1112 has a first end 11121 and a second end 11122. The first drainage tube 1111 is connected to the first end 11121 of the switching member 1112 through the first through hole 11212 from the back side 1121b' to the front side 1121a' of the board body 1121'. The second drainage tube 1113 is connected to the second end 11122 of the switching member 1112 and extends through the second through hole 11213 to the back side 1121b' of the board body 1121' and then extends through the third through hole 11214. The front surface 1121a' of the board body 1121', wherein the flow rate measuring module 122 is detachably fixed to the body fluid drainage tube 111 at a portion of the second drainage tube 1113 on the back side 1121b' of the board body 1121' to monitor the flow rate of the liquid in the tube, thereby avoiding the fixation and operation of the flow detecting module 122 and the drainage setting of the body fluid drainage tube 111 at the front side 1121a of the board body 1121' and the adjustment of the collecting member 113. Observing mutual interference and introducing the body fluid drainage tube 111 is more stably fixed to the main board 112'.

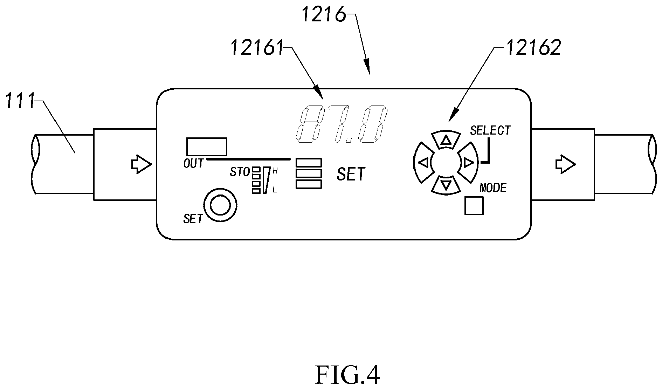

Referring to FIGs. 4-5, the interface module 1216 according to the aforementioned first preferred embodiment of the present invention is preferably disposed outside the flow rate measuring module 122 to facilitate the related setting or operation through the interface module 1216 when the body fluid drainage tube 111 and the flow rate measuring module 122 are detachably coupled, and can also be conveniently used for subsequent adjustment, inspection, or other operations using the interface module 1216. The interface module 1216' according to an alternative embodiment of the first preferred embodiment of the present invention includes the display unit 12161' and the input unit 12162' being disposed outside the host 121. Although this embodiment does not have the aforementioned advantages, since it is not required to be disposed on the flow rate measuring module 122 which is generally small in size, the flow rate measuring module 122 can be further thinned or reduced, or the manufacturing cost can thus be reduced, and the device can be updated modularly. It should be understood by those skilled in the art that, in practical applications, the interface module 1216 can also be disposed in other manners, for example, an external device is independently fixed on the frame and configured as a remote-control device, etc. The embodiments and the scope of the invention are not limited thereto.

In addition, the host 121 may communicate with an external device through the wireless communication module 1214 and/or the wired communication module 1215, that is to connect external devices wiredly and/or wirelessly and is not limited to one-to-one connection. Therefore, in practice, a plurality of the body fluid drainage monitoring members 12 can be monitored remotely or proximally by an external device, such as a computer, a tablet, a mobile phone, etc., which is equivalent to being simultaneously monitor the drainage condition of several patients. And since the implementation of the monitoring is not limited to the position of each of the body fluid drainage monitors 12, the medical personnel responsible for monitoring can monitor the drainage conditions of several patients simultaneously and comprehensively without walking back and forth in each ward or hospital bed. At the same time, the medical personnel can also manage, set, and view each of the body fluid drainage monitoring members 12 remotely or proximally, separately or in batches, manually or automatically, and even more management application through the programs developed for external devices. In addition, the external device can analyze, store, and automatically alert the obtained drainage status information. In addition to having clinically immediate functions, it also contributes to post-mortem analysis and academic research purposes. This will improve the efficiency and effectiveness of medical personnel and reduce the workload of medical personnel, thereby reducing the risk of medical personnel making mistakes in related or other work, and also helping researchers to obtain relevant data quickly and completely.

It is worth mentioning that the wireless communication module 1214 is implemented as a wireless high-fidelity (WiFi) signal transmission unit in this embodiment, for communicating with other devices through the WiFi signal, so that the body fluid drainage monitoring member 12 has the ability to communicate wirelessly with other devices or networks. It should be understood by those skilled in the art that the communication herein is not limited to direct communication. In other words, the wireless communication module 1214 can also be implemented in an actual application to communicate with other external devices or wireless devices indirectly through a WiFi signal, for example, through a radio signal booster, router enhances or relays the signal to connect to the communication target device or to communicate with other or external devices via the internal and external networks. In addition to WiFi, its wireless signal can be replaced or added as Bluetooth, Bluetooth Low Energy (BLE), Near Field Communication (NFC), Infrared Data Association Standard (IrDA), GSM Signal, CDMA Signal, TD-CDMA. Signals, 3G signals, 4G signals, 5G signals, Zigbee, Worldwide Interoperability for Microwave Access (WiMAX), and other means of data transmission known to those skilled in the art.

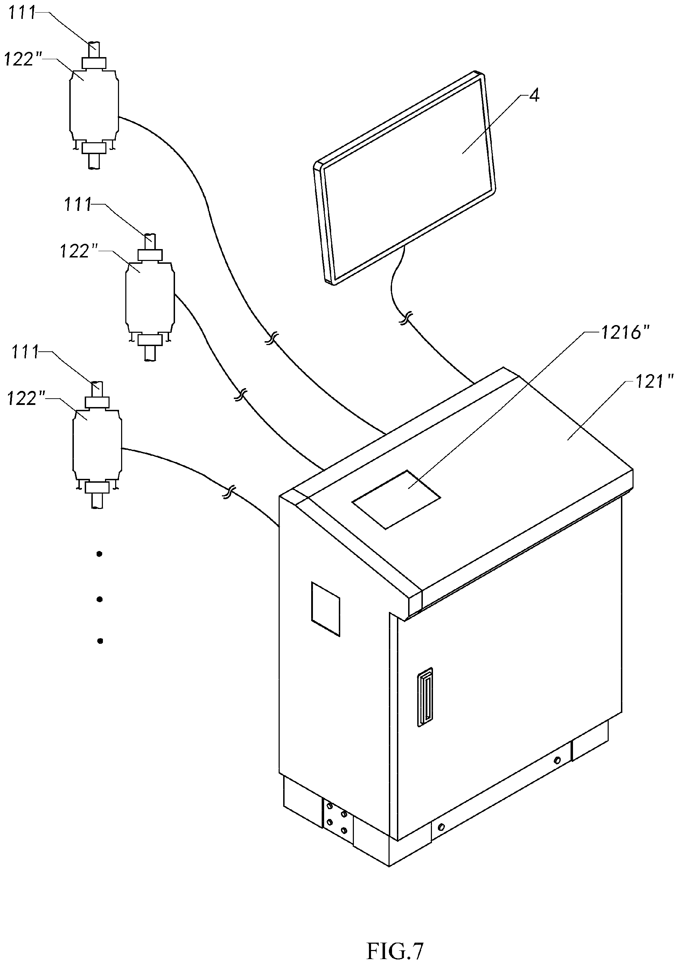

Referring to FIG. 7, in accordance with a second preferred embodiment of the present invention, the body fluid drainage monitoring member 12 can also be implemented to include a host 121" and a plurality of flow rate measuring modules 122" electrically connected to the host 121". The flow rate measuring modules 122" can be managed, set, and monitored through the same host 121", thereby avoiding problems that may be caused by multiple hosts, such as high cost, space occupation, etc., also saves the mental energy consumption of the person walking around the different host 121" for managing, setting, and monitoring the plurality of the flow rate measuring modules 122". In addition, the host 121" is also allowed to be electrically connected to an external interface device 4, so that it is more flexible in use and construction. For example, the host 121" can be placed in a ward for simultaneously managing, setting, monitoring one or more flow rate measuring modules 122" disposed at different beds of the ward, and then the external interface device 4 is extendedly disposed on the outer wall of the ward or the nurse station. The medical person can manage, set, and/or monitor each of the flow rate measuring modules 122" without entering the ward. The external interface device 4 can be embodied as a pure display device, a touch display, a tablet computer or other terminals. The specific implementation manner and the scope of the present invention are not limited thereto. Further, in this embodiment, the host 121", the flow rate measuring module 122", and the connection between the external interface devices 4 uses physical lines. For example, the connection between the former two can use 4∼20mA analog signal lines, etc., but this is only an implementation manner, in practical applications or according to other variants or in alternative embodiments, some or all of the connections may be replaced with wireless connections, such as WiFi, Bluetooth, etc., and the specific embodiments and the scope of the present invention are not limited thereto.

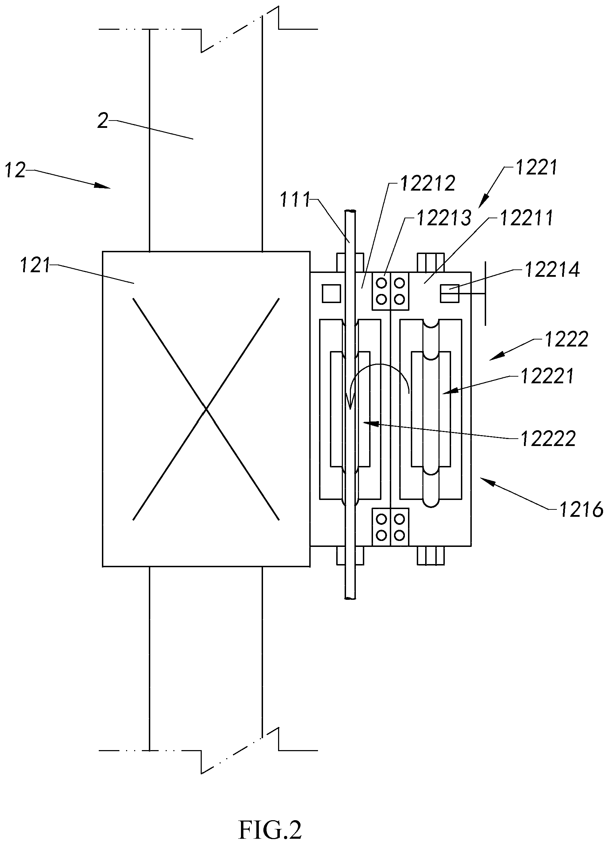

Referring to FIG. 2-3, the flow rate measuring module 122 includes a conduit fastening member 1221 configured to detachably couple to the body fluid drainage tube 111 and a flow rate measuring member 1222 disposed on the conduit fastening member 1221. The conduit fastening member 1221 includes a first fastening member 12211, a second fastening member 12212, a connecting member 12213, and a fastening member 12214. The first fastening member 12211 and the second fastening member 12212 are adjacent at one side and are openably connected by the connecting member 12213, thereby allowing the fastening member 12214 disposed on the other side of the first fastening member 12211 and the second fastening member 12212 latching or locking the first fastening member 12211 and the second fastening member 12212 when the first fastening member 12211 and the second fastening member 12212 are opposite to each other, that is in the closed state. When the body fluid drainage tube 111 needs to be fixed, the fastener 12214 is released to open the first fastening member 12211 and the second fastening member 12212, thereby allowing the body fluid drainage tube 111 to be placed in the recessed structure therein, and then the first fastening member 12211 and the second fastening member 12212 are closed and locked by the fastening member 12214, so that the body fluid drainage tube 111 is fastened by the first fastening member 12211 and the second fastening member 12212.

The flow detecting component 1222 includes a first supersonic transceiving unit 12221 and a second supersonic transceiving unit 12222 disposed on the inner side of the first fastening member 12211 and the second fastening member 12212, respectively. Therefore, when the first fastening member 12211 and the second fastening member 12212 fasten the body fluid drainage tube 111 therein, the supersonic wave can be transmitted and received through the body fluid drainage tube 111 to detect the drainage condition in the body fluid drainage tube 111.

It is worth mentioning that the status information obtained by the flow rate measuring member 1222 of the body fluid drainage monitoring member 12 is a real-time digital information. In comparison with the medical staff by using the conventional dropper observation method, it does not take time to observe and confirm the scale and calculate the flow rate. It also eliminates the risk of misjudgment of the drainage condition due to personnel observation or calculation errors and the risk of leaving the wrong research information afterwards, which can reduce the risk of clinical misdiagnosis and academic research errors.

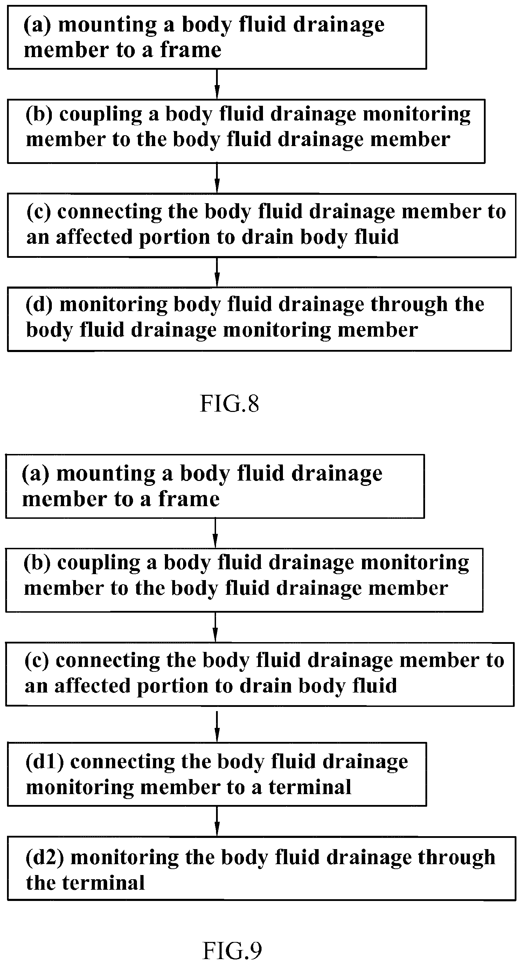

Referring to Figures 8-9, in accordance with a preferred embodiment of the present invention, the present invention also provides a body fluid drainage method including the following steps:
(a) mounting a body fluid drainage member to a frame;
(b) coupling a body fluid drainage monitoring member to the body fluid drainage member;
(c) connecting the body fluid drainage member to an affected portion to drain body fluid; and
(d) monitoring body fluid drainage through the body fluid drainage monitoring member.

wherein the step (a) is performed by mounting body fluid drainage member to a frame such that the body fluid drainage member is fixed at a specific height relative to an affected portion having a fixed height. Secondly, the step (b) coupling a body fluid drainage monitoring member to the body fluid drainage member to allow the user to monitor the drainage condition of the body fluid drainage device through the body fluid drainage monitoring member. Furthermore, when performing the step (c), the user can connect the body fluid drainage member to an affected portion by direct or indirect means to introduce fluid, and then perform the step (d) using the body fluid drainage monitoring member to monitor the drainage condition to implement the application of the body fluid drainage device of the present invention.

Further, the step (d) may further include the following steps:
(d1) connecting the body fluid drainage monitoring member to a terminal;
(d2) monitoring the body fluid drainage through the terminal.

In the step (d1), the body fluid drainage monitoring member is connected to a terminal, that is to connect the body fluid drainage monitoring member to an external device by wiredly and/or wirelessly and is not limited to a one-to-one connection. The step (d2), monitoring the drainage state through the terminal, is to further monitor one or more of the body fluid drainage by an external device, such as a computer, a tablet, a mobile phone, etc., at the same time. The monitor is implemented to achieve the aforementioned or other various modes of application. It is worth mentioning that the monitoring refers not only to passive monitoring and active measurement of drainage state data, but also includes the foregoing or other various application modes, such as analyzing record data, setting parameters, opening and closing monitoring, managing data, providing warnings. The data is obtained afterwards, and the scope of the present invention is not limited thereto.

## Claims

1. A body fluid drainage device adapted to installation on a frame for draining body fluid of an affected portion, comprising:
a body fluid drainage member configured to drain body fluid of the affected portion; and
a body fluid drainage monitoring member detachably coupled to the body fluid drainage member and configured to monitor body fluid drainage.

2. The body fluid drainage device as claimed in claim 1, wherein the body fluid drainage member comprises a body fluid drainage tube, and the body fluid drainage monitoring member is detachably coupled to the body fluid drainage tube and configured to monitor the body fluid drainage.

3. The body fluid drainage device as claimed in claim 2, wherein the body fluid drainage member further comprises a main board and a collecting member movably disposed on the main board, wherein the body fluid drainage tube is connected to the collecting member for draining the body fluid to the collecting member, wherein the main board is adapted to installation on the frame near the affected portion so that the collecting member is movably disposed at a height corresponding to the affected portion.

4. The body fluid drainage device as claimed in claim 3, wherein the body fluid drainage tube comprises a first drainage tube connected to the affected portion, a switching member and a second drainage tube, wherein the switching member has a first end connected to the first drainage tube, a second end connected to the second drainage tube, a third end adapted to connection of an external equipment and a switch configured to switch connection between the first end, the second end and the third end or between any two of the first end, the second end and the third end.

5. The body fluid drainage device as claimed in claim 3, wherein the main board comprises a board body, a hanging member connected to the board body and configured to hang on the frame near the affected portion and a first movably-fastening member configured to movably fasten the collecting member, wherein the board body has a front surface, a back surface and a scale formed on the front surface and configured to indicate a position of the collecting member, wherein the body fluid member further comprises a body fluid storage member detachably disposed on the plate and connected to the colleting member for storing and depositing the body fluid.

6. The body fluid drainage device as claimed in claim 5, wherein the collecting member comprises a main body configured to collect body fluid, an one-way discharge valve configured to purge gas in the main body and a second movably fastening member connected to the main body and configured to movably coupled to the first movably fastening member so as to movably fasten the collecting member to the board body, wherein the collecting member has a body fluid inlet connected to the body fluid drainage tube, an exit connected to the one-way valve and a body fluid outlet, wherein the collecting member further comprises a body fluid outflow tube connected to the body fluid outlet and the body fluid storage member.

7. The body fluid drainage device as claimed in claim 5, wherein the board body includes at least one or more through holes through which the body fluid drainage tube extends so as to be arranged on a front side and a back side of the board body.

8. The body fluid drainage device as claimed in claim 2, wherein the body fluid drainage monitoring member comprises a host and a flow rate measuring module connected to the host, wherein the flow rate measuring module is detachably coupled to the body fluid drainage tube for monitoring the body fluid drainage.

9. The body fluid drainage device as claimed in claim 8, wherein the host comprises a power supply configured to supply electrical power, a processing module electrically connected to the power supply for calculation, a data storage module electrically connected to the processing module for storing data, and an interface module electrically connected to the processing module for inputting and outputting data.

10. The body fluid drainage device as claimed in claim 9, wherein the host further comprises a fastening member configured to detachably fasten the host to the frame near the affected portion.

11. The body fluid drainage device as claimed in claim 10, wherein the host further comprises an alarm module configured to alarm according to a control of the processing module.

12. The body fluid drainage device as claimed in claim 9, wherein the host further comprises a wired communication module or a wireless communication module electrically connected to the processing module for wired or wireless communication.

13. The body fluid drainage device as claimed in claim 9, wherein the flow rate measuring module comprises a conduit fastening member configured to detachably coupled to the body fluid drainage tube and a flow rate measuring member disposed in the conduit fastening member, wherein the flow rate measuring member comprises a first supersonic transceiver and a second supersonic transceiver corresponding to the first supersonic transceiver, the first supersonic transceiver and the second supersonic transceiver transmit supersonic waves to measure a flow rate of the body fluid in the body fluid drainage tube.

14. A body fluid draining method, comprising:
(a) mounting a body fluid drainage member to a frame;
(b) coupling a body fluid drainage monitoring member to the body fluid drainage member;
(c) connecting the body fluid drainage member to an affected portion to drain body fluid; and
(d) monitoring body fluid drainage through the body fluid drainage monitoring member.

15. The body fluid draining method as claimed in claim 14, the step (d) further comprising:
(d1) connecting the body fluid drainage monitoring member to a terminal; and
(d2) monitoring the body fluid drainage through the terminal.
